# EUROPEAN PATENT APPLICATION

(11) **EP 4 245 307 A1**
(43) Date of publication of application: **20.09.2023**
(21) Application number: 21892295.3
(22) Date of filing: 09.11.2021
(51) Int. Cl.: A61K 35/28, A61K 38/19, A61P 21/00, C07K 14/52, C07K 14/47, C12N 5/0775, A23L 33/10, A23L 33/17

(54) **COMPOSITION FOR PREVENTING OR TREATING MUSCLE DISEASES, CONTAINING STEM CELLS IN WHICH GRO-? IS EXPRESSED OR GRO-?**

(30) Priority: 13.11.2020 KR 20200152280
(71) Applicant: Samsung Life Public Welfare Foundation, Seoul 04348 (KR); Encell Co., Ltd., Gangnam-gu, Seoul 06072 (KR)
(72) Inventor: CHANG, Jong Wook, Seoul 06351 (KR); JEON, Hong Bae, Seoul 05531 (KR); PARK, Sang Eon, Seongnam-si, Gyeonggi-do 13500 (KR); KWON, Soojin, Namyangju-si, Gyeonggi-do 12124 (KR)
(74) Representative: Patentanwälte Olbricht Buchhold Keulertz
(86) International application number: PCT/KR2021/016262
(87) International publication number: WO 2022/103128

(57) **Abstract**

Provided is a composition for preventing or treating a muscle disease, the composition including: a stem cell expressing growth-regulated oncogene-alpha (GRO-α); and/or GRO-α, wherein GRO-α inhibits death of muscle cells or skeletal muscle myoblasts. Thus, the composition can be used as an agent for prevention or treatment of various muscle diseases.

## Description

### Technical Field

The present application claims priority to Korean Patent Application No. 10-2020-0152280, filed on November 13, 2020, the disclosure of which is incorporated by reference in its entirety.

The present application relates to a composition for preventing or treating a muscle disease, the composition including growth-regulated oncogene-alpha (GRO-α) or a stem cell expressing GRO-α.

### Background Art

Mesenchymal stem cells are multipotent cells and can differentiate into bones, cartilages, muscles, fats, and fibroblasts. Recently, studies to utilize the mesenchymal stem cells as therapeutic agents for various diseases have been actively conducted.

However, muscle diseases are mostly rare diseases that ordinary people cannot easily experience, and thus people are not much aware of muscle diseases and the history of research on muscle diseases is short. Also, up to date, there are few therapeutic agents for muscle diseases. As in the general cases with rare diseases, symptoms of muscle diseases progress rapidly and are severe, and depending on types of the muscle diseases, symptoms and disease intensity vary and may appear in a complex form due to nervous system problems in neurons or spinal cord. Patients with muscle diseases are unable to do daily lives alone as the disease progresses, and painfully suffer in reality from no fundamental therapeutic agents available at all.

Therefore, cell therapy agents need to be developed for more fundamental treatment of muscle diseases.

### Disclosure

### Technical Problem

An aspect is to provide a cell death inhibitor including: a mesenchymal stem cell genetically engineered to secrete growth-regulated oncogene-alpha (GRO-α) or overexpress GRO-α compared to a parental cell; or GRO-α.

Another aspect is to provide a pharmaceutical composition for preventing or treating a muscle disease, the pharmaceutical composition including: a mesenchymal stem cell genetically engineered to secrete GRO-α or overexpress GRO-α compared to a parental cell; or GRO-α.

Another aspect is to provide a pharmaceutical composition for preventing or treating a muscle disease, the pharmaceutical composition including GRO-α as an active ingredient.

Another aspect is to provide a health functional food composition for preventing or treating a muscle disease, the health functional food composition including GRO-α as an active ingredient.

Another aspect is to provide a cell therapy agent for treating a muscle disease, the cell therapy agent including: a mesenchymal stem cell genetically engineered to secrete GRO-α or overexpress GRO-α compared to a parental cell; or GRO-α.

Another aspect is to provide a method of preventing or treating a muscle disease, the method including administering a pharmaceutically effective amount of a mesenchymal stem cell, which is genetically engineered to secrete GRO-α or overexpress GRO-α compared to a parent cell, or GRO-α, to a subject in need thereof.

Another aspect is to provide use of a composition for treating a muscle disease, the composition including: a mesenchymal stem cell genetically engineered to secrete GRO-α or overexpress GRO-α compared to a parent cell; or GRO-α.

Another aspect is to provide use of a composition for preparing a therapeutic agent for a muscle disease, the composition including: a mesenchymal stem cell genetically engineered to secrete GRO-α or overexpress GRO-α compared to a parent cell; or GRO-α.

### Technical Solution

An aspect provides a cell death inhibitor including: a mesenchymal stem cell genetically engineered to secrete growth-regulated oncogene-alpha (GRO-α) or overexpress GRO-α compared to a parental cell; or GRO-α. Another aspect provides a method of inhibiting cell death, the method including: *in vitro* contacting of a mesenchymal stem cell, which is genetically engineered to secrete GRO-α or overexpress GRO-α compared to a parental cell, or GRO-α, to a cell; or *in vivo* administering of a mesenchymal stem cell, which is genetically engineered to secrete GRO-α or overexpress GRO-α compared to a parental cell, or GRO-α, to an experimental animal. In an embodiment, the cell death inhibitor may include: a mesenchymal stem cell genetically engineered to secrete GRO-α or overexpress GRO-α compared to a parent cell; and GRO-α. In an embodiment, when cell death-induced muscle cells by lovastatin were co-cultured with mesenchymal stem cells of the placenta or umbilical cord, an increase in expression of GRO-α protein is confirmed. Therefore, it is considered that the GRO-α protein secreted from mesenchymal stem cells may be able to inhibit cell death.

The term "growth-regulated oncogene-alpha (GRO-α)" as used in the present specification is one of the chemokine subfamily and plays an essential role in recruitment and activation of neutrophils in response to tissue damage and microbial infection. This term may be used interchangeably with chemokine (C-X-C motif) ligand 1 (CXCL).

The term "genetic engineering" or "genetically engineered" as used in the present specification refers to: the act of introducing one or more genetic modifications into a cell; or to a cell prepared thereby. For example, the mesenchymal stem cell or a host cell may be genetically engineered to increase expression or activity of GRO-α or an active fragment thereof, and for example, may include an exogenous gene which encodes GRO-α or an active fragment thereof. The increased activity may refer to, compared to activity of an endogenous protein or enzyme absent or included in a given, non-genetically engineered parent cell (e.g., a wild-type cell), higher activity of the same type of protein or enzyme. The exogenous gene may be expressed increasingly in the mesenchymal stem cell or the host cell, compared to a parent cell thereof, in an amount sufficiently enough to increase the activity of the aforementioned protein. The exogenous gene may be introduced into the parent cell through an expression vector. Also, the exogenous gene may be introduced in the form of a linear polynucleotide into the parent cell. Also, the exogenous gene may be expressed from an expression vector (e.g., a plasmid) in a cell. Also, the exogenous gene may be inserted into a genetic material (e.g., a chromosome) and then expressed in a cell, for stable expression.

Another aspect provides a pharmaceutical composition for preventing or treating a muscle disease, the pharmaceutical composition including: a mesenchymal stem cell genetically engineered to secrete GRO-α or overexpress GRO-α compared to a parental cell; or GRO-α. Another aspect provides a method of preventing or treating a muscle disease, the method including administering a pharmaceutically effective amount of a mesenchymal stem cell, which is genetically engineered to secrete GRO-α or overexpress GRO-α compared to a parent cell, or GRO-α, to a subject in need thereof. Details on GRO-α or the mesenchymal stem cell genetically engineered to secrete GRO-α or overexpress GRO-α compared to a parental cell are the same as described herein.

In an embodiment, it is confirmed that, when cell death-induced muscle cells by lovastatin are treated with GRO-α, the cell death by apoptosis and necrosis is inhibited, and the cell proliferation and cell viability are accordingly increased. In addition, it is confirmed that, when an MDX mouse as a mouse model for duchenne muscular dystrophy is treated with the mesenchymal stem cells or the GRO-α protein, the activity of creatine kinase is inhibited, and the expression of necrosis markers, i.e., phospho-MLKL and phospho-RIP3, is significantly reduced. Accordingly, the GRO-α protein can be used for the prevention or inhibition of muscle diseases by inhibiting the cell death of muscle cells and increasing the cell viability.

In an embodiment, the mesenchymal stem cells may be derived from umbilical cord, umbilical cord blood, bone marrow, placenta, adipose, and the like. The umbilical cord may refer to a tube that connects the belly of a mammalian fetus to the body of a mother so that the mammalian fetus can grow in the placenta. In general, the umbilical cord may refer to a tissue composed of three vessels, i.e., two umbilical arteries and one umbilical vein, surrounded by Wharton's jelly. Therefore, in an embodiment, the mesenchymal stem cells may be derived from the umbilical cord or umbilical cord blood.

The term "muscle disease" as used in the present specification includes any disease related to muscle wasting or muscle damage. In detail, a muscle wasting-related disease refers to a disease or condition accompanied by symptoms such as gradual loss of muscle mass and the like. The muscle wasting may be caused by various factors including: genetic predispositions; age-related diseases such as hypertension, impaired glucose tolerance, diabetes, obesity, dyslipidemia, atherosclerosis, and cardiovascular disease; chronic diseases such as cancers, autoimmune diseases, infectious diseases, AIDS, chronic inflammatory diseases, arthritis, malnutrition, renal diseases, chronic obstructive pulmonary diseases, pulmonary emphysema, rachitis, chronic lower spine pain, peripheral nerve injury, central nerve injury, and chemical injury; conditions such as long-term immobilization, ineffectualness-like conditions such as bone fractur or trauma, and post-surgery bed rest; and progressive decrease in skeletal muscle mass and strength caused by aging processes. The muscle wasting-related disease may cause a weakened physical condition, resulting in deterioration of health conditions or health conditions of incapacitated physical performance. In addition, the muscle injury refers to any injury in muscle tissue, and may be caused by physical trauma to muscle tissue due to accidents, sports injury, endocrine gland disorders, diseases, wound, or surgical operation. The muscle disease may be, for example, sprain, strain, spasm, tendinitis, muscle cancer, myositis, rhabdomyolysis, muscular atrophy, atony, muscular dystrophy, muscular degeneration, myasthenia, dystrophinopathy, myopathy, sacopenia, Charot-Marie-Tooth disease, and the like. The pharmaceutical composition according to an aspect has an effect of preventing or treating various muscle diseases by inhibiting the death of muscle cells or skeletal muscle myoblasts.

Another aspect provides a pharmaceutical composition for preventing or treating a muscle disease, the pharmaceutical composition including GRO-α as an active ingredient. Details on GRO-α and the muscle disease are the same as described herein. In an embodiment, GRO-α may be separated from a mesenchymal stem cell.

In one or more embodiments, the pharmaceutical composition may further include a mesenchymal stem cell. The mesenchymal stem cell may secrete GRO-α or an active fragment thereof, or may be genetically engineered to secrete GRO-α or an active fragment thereof. In detail, the mesenchymal stem cell may be modified by insertion or injection of DNA in cell culture by a method of altering, enhancing, or supplementing cell functions for induction to structural or therapeutic purposes. In this regard, GRO-α and the mesenchymal stem cells may be co-administered.

The pharmaceutical composition for preventing or treating a muscle disease according to an aspect may have dosage forms, each according to methods known in the art, including a formulation for oral administration such as a powder, a granule, a tablet, a capsule, a suspension, an emulsion, a syrup, an aerosol, and the like, an external preparation formulation, or a sterile injection solution formulation. For such dosage forms, a suitable carrier, excipient, or diluent commonly used in the preparation of a pharmaceutical composition may be included.

For use as the carrier, excipient, and diluent, various compounds or mixture including lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, or mineral oil may be used.

When formulated, a commonly used diluent or excipient, such as a filler, an expander, a binder, a wetting agent, an disintegrant, a surfactant, and the like, may be used for preparation.

A solid preparation for oral administration may be prepared by mixing the beans extract with at least one excipient, such as starch, calcium carbonate, sucrose, lactose, gelatin, and the like. Also, in addition to a simple excipient, lubricants, such as magnesium stearate and talc, may be used.

For use as a liquid formulation for oral administration, a suspension, an oral liquid, an emulsion, a syrup, and the like may be used. In addition to water and liquid paraffin, which are simple diluents commonly used, various excipients, such as a wetting agent, a sweetening agent, a flavoring agent, a preservative, and the like, may be included.

For use as a formulation for parenteral administration, a sterile solution, a non-aqueous agent, a suspension, an emulsion, a freeze-dried agent, a suppository, and the like may be included. Examples of the non-aqueous agent and the suspension are propylene glycol, polyethylene glycol, plant oil such as olive oil, and injectable ester such as ethyl oleate. For use as a base agent for the suppository, witepsol, macrogol, tween 61, cacao butter, laurin butter, glycerol, gelatin, or the like may be used.

A preferable dosage of the pharmaceutical composition for preventing or treating a muscle disease according to an aspect may vary depending on a condition of a patient, a body weight of a patient, severity of a disease, a drug form, an administration route, and an administration period, but may be appropriately selected by those skilled in the art. However, for a desirable effect, the dosage may be in a range of about 0.0001 mg/kg to about 2,000 mg/kg per day, preferably, about 0.001 mg/kg to about 2,000 mg/kg per day. The administration may be performed once a day or several times a day. However, the scope of the present disclosure is not limited by the dosage.

The pharmaceutical composition for preventing or treating a muscle disease according to an aspect may be administered to mammals, such as rats, mice, livestock, humans, and the like, via various routes. All administration methods may be, for example, performed orally or rectally or by an intravenous, intramuscular, subcutaneous, intrauterine dural, or intracerebroventricular injection.

Another aspect provides a health functional food composition for preventing or ameliorating a muscle disease, the health function food composition including GRO-α as an active ingredient. Details on GRO-α and the muscle disease are the same as described herein.

In the health functional food composition for preventing or ameliorating a muscle disease according to an aspect, when the compound is used as an additive of a health functional food, the compound may be added as it is or used in combination with other foods or food ingredients, appropriately according to methods known in the art. Here, a mixing amount of the active ingredient may be appropriately determined according to each purpose of use, such as prevention, health care, or treatment.

The health functional food may be in any form of a powder, a granule, a pill, a tablet, or a capsule, as well as a general food or beverage form.

Types of the general food are not particularly limited, and examples of the general food to which the additives can be added are meat, a sausage, a bread, a chocolate, a candy, a snack, a confectionery, a pizza, a ramen, other noodles, a gum, a dairy product including ice cream, various soups, a beverage, a tea, a drink, an alcoholic beverage, a vitamin complex, and the like, and may include all types of foods in the ordinary sense.

In general, when preparing a food or a beverage, the additives may be added in an amount of 15 parts by weight or less, preferably, 10 parts by weight or less, based on 100 parts by weight of the raw material. However, in the case of long-term intake for the purpose of health and hygiene or health care, the amount may be less than the ranges above. Also, since there is no problem in terms of safety in that fractions from natural products are used, the amount greater than the ranges above may be used.

Among the health functional food according to an aspect, a beverage may contain, as additive ingredients, various flavoring agents or natural carbohydrates as in general beverages. Examples of the natural carbohydrate may include a monosaccharide, such as glucose and fructose, a disaccharide, such as maltose and sucrose, a polysaccharide, such as dextrin and cyclodextrin, and sugar alcohol, such as xylitol, sorbitol, and erythriotol. For use as the sweetening agent, a natural sweetening agent, such as thaumatin and a stevia extract, a synthetic sweetening agent, such as saccharin, aspartame, and the like may be used. A ratio of the natural carbohydrate in the beverage may be in a range of about 0.01 g to about 0.04 g, preferably, about 0.02 g to about 0.03 g, per 100 mL of the beverage according to the present disclosure.

Furthermore, the health functional food composition for preventing or ameliorating a muscle disease according to an aspect may include various nutrients, vitamins, electrolytes, flavors, coloring agents, pectic acid and a salt thereof, alginic acid and a salt thereof, organic acids, protective colloidal thickeners, pH adjusters, stabilizers, preservatives, glycerin, alcohol, carbonizing agent used in carbonated beverages. Moreover, the composition for sleep improvement of the present disclosure may include fruit pulps for preparing a natural fruit juice, a fruit juice beverage, and a vegetable juice. Such components may be used independently or in combination. A ratio of these additives is not limited, but may be generally selected from the range of about 0.01 parts by weight to about 0.1 parts by weight based on 100 parts by weight of the health functional food composition of the present disclosure.

Another aspect provides a cell therapy agent for treating a muscle disease, the cell therapy agent including: a mesenchymal stem cell genetically engineered to secrete GRO-α or overexpress GRO-α compared to a parental cell; or GRO-α. Details on GRO-α and the mesenchymal stem cell genetically engineered to secrete GRO-α or overexpress GRO-α compared to a parental cell are the same as described herein.

The term, "cell therapy agent" as used in the present specification refers to a drug for the purpose of treatment, diagnosis, and prevention through a series of actions that change biological characteristics of cells by *in vitro* proliferation, selection, or various methods using living autologous, allogenic, or xenogenic cells, so as to restore functions of cells and tissues.

As described above, by secreting GRO-α, the mesenchymal stem cell according to an aspect inhibits the cell death of muscles cells and promotes the cell proliferation, and thus can be used for the prevention or treatment of a muscle disease.

### Advantageous Effects

The mesenchymal stem cell or GRO-α according to an aspect inhibits the death of muscle cells or skeletal muscle myoblasts, and thus can be used as an agent for preventing or treating various muscle diseases including sprain, strain, cramp, and the like.

### Description of Drawings

FIG. 1A shows results of confirming anti-cell death effects of human placenta-derived mesenchymal stem cells (PL-MSCs) and human Wharton's Jelly-derived mesenchymal stem cells (WJ-MSCs) in skeletal muscle cells.
FIG. 1B shows results of expression of cell death markers by PL-MSCs and WJ-MSCs as confirmed by western blotting.
FIG. 1C shows a graph of quantifying the expression of cell death markers by PL-MSCs and WJ-MSCs.
FIG. 2A shows results of confirming anti-necroptotic effects of PL-MSCs and WJ-MSCs in skeletal muscle cells.
FIG. 2B shows a graph of quantifying the states of necrotic and apoptic cells in PL-MSCs and WJ-MSCs.
FIG. 3 shows results of analyzing proteins commonly expressed in PL-MSCs and WJ-MSCs.
FIG. 4A shows the results of cell morphology after C2C12 cells that were induced to death by lovastatin treatment are treated with GRO-α at different concentrations.
FIG. 4B shows the results of confirming cell viability after C2C12 cells death-induced by lovastatin treatment are treated with GRO-α at different concentrations.
FIG. 4C shows the results of expression of necrosis markers as confirmed through western blotting after C2C12 cells death-induced by lovastatin treatment are treated with GRO-α at different concentrations.
FIG. 4D shows a graph of quantifying the expression of necrosis markers after C2C12 cells death-induced by lovastatin treatment are treated with GRO-α at different concentrations.
FIG. 5A shows the results of cell morphology after C2C12 cells that were induced to death by lovastatin treatment are treated with GSK'872 and GRO-α individually or in combination.
FIG. 5B shows the results of confirming cell viability after C2C12 cells death-induced by lovastatin treatment are treated with GSK'872 and GRO-α individually or in combination.
FIG. 5C shows results of expression of necrosis markers as confirmed through western blotting after C2C12 cells death-induced by lovastatin treatment are treated with GSK'872 and GRO-α individually or in combination.
FIG. 5D shows a graph of quantifying the expression of necrosis markers after C2C12 cells death-induced by lovastatin treatment are treated with GSK'872 and GRO-α individually or in combination.
FIG. 6A shows a graph of creatine kinase activity in the blood of Duchenne muscular dystrophy mouse model injected with WJ-MSC and GRO-α.
FIG. 6B shows the results of measuring expression levels of phospho-MLKL at a protein level in muscles of Duchenne muscular dystrophy mouse model injected with WJ-MSC and GRO-α.
FIG. 6C shows the results of measuring expression levels of phospho-RIP3 at a protein level in muscles of Duchenne muscular dystrophy mouse model injected with WJ-MSC and GRO-α.

### Best Mode

### Mode for Invention

Hereinafter, preferable Examples are presented to help understanding of the present disclosure. However, the following examples are only presented for easier understanding of the present disclosure, and the contents of the present disclosure are not limited by the following examples.

### [Examples]

### Example 1. Confirmation of anti-cell death effects of human placenta-derived mesenchymal stem cells and human Wharton's Jelly-derived mesenchymal stem cells in skeletal muscle myoblasts

Inhibitory effects of human placenta-derived mesenchymal stem cells (PL-MSCs) and human Wharton's Jelly-derived mesenchymal stem cells (WJ-MSCs) on death of skeletal muscle myoblasts were confirmed.

The human PL-MSCs and WJ-MSCs were prepared after securing the umbilical cord and placenta through a collaborative study with the Department of Obstetrics and Gynecology at Samsung Seoul Hospital according to the IRB protocol (IRB# 2015-09-023-003) approved thereby. Then, separation of the mesenchymal stem cells was carried out according to the previously reported protocol. In detail, after the umbilical cord was cut to a length of 3 cm to 4 cm, tissues of the umbilical cord were cut into small pieces and treated with a collagenase solution (Gibco, USA) for 60 minutes to 90 minutes to decompose the extracellular matrix. Then, 0.25 % trypsin (Gibco, USA) was added thereto to additionally induce decomposition for 30 minutes at 37 °C. Afterwards, fetal bovine serum (FBS; Biowest, USA) was added, and the cells obtained by centrifugation at 1,000 g for 10 minutes were cultured in a Dulbecco Modified Eagle Medium (DMEM; Biowest, USA) supplemented with 20 % FBS and 1 % penicillin-streptomycin (Gibco, USA) at 37 °C in a 5 % CO₂ environment, and WJ-MSCs at passages 5 and 6 were used for the experiment. Next, to induce death of skeletal muscle myoblasts, skeletal muscle myoblasts of a C2C12 mouse were treated with 10 µm of lovastatin and cultured for 3 hours. Accordingly, the cell death was induced. Afterwards, PL-MSCs and WJ-MSCs were co-cultured with the mouse skeletal muscle myoblasts, respectively. As a control, the cell death-induced mouse skeletal muscle myoblasts were used.

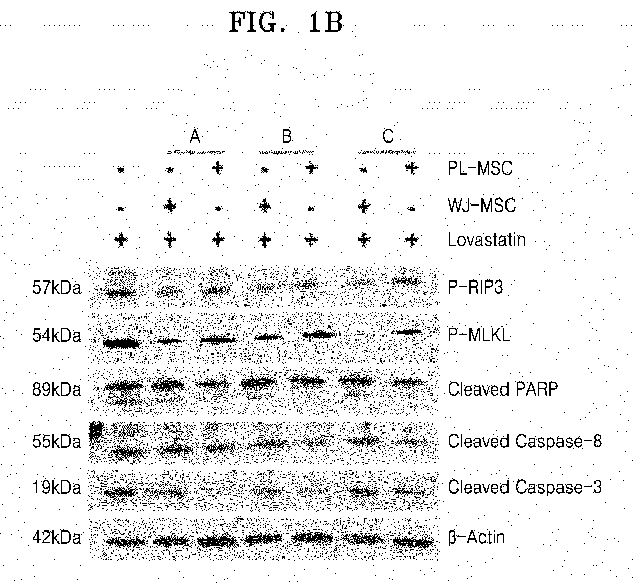

To confirm the inhibitory effects of the PL-MSCs and WJ-MSCs on the cell death, apoptosis markers and necrosis markers expressed in the mouse skeletal muscle myoblasts were confirmed by western blotting. In detail, after collecting the cells and breaking the cell membrane by using a buffer in which RIPA buffer and urea buffer were mixed at a ratio of 1:1, centrifugation was performed thereon, and the supernatant thus obtained was collected and subjected to the Bradford assay (Bio-rad, USA) to quantify proteins in the cells. After loading 20 µg of the proteins of from each sample on a gel, electrophoresis was performed on the gel by using SDS. After transferring the proteins to a polyvinylidene fluoride (PVDF) membrane, a 1 × tris-buffered saline with 0.1 % Tween 20 (TBST) solution to which 5 % skimmed milk (BD Difco, USA) was added, followed by culturing at room temperature for 1 hour. Afterwards, the proteins were treated with PARP (1:1000, Cell signaling, USA), cleaved caspase-3 (1:1000, Cell signaling, USA), phospho-RIP3 (1:1000, Cell signaling, USA), and phospho-MLKL (1:1000, Cell signaling, USA) and beta actin (1:5000; Santa Cruz, USA) antibodies, followed by culturing overnight at 4 °C. Next, the proteins were treated with secondary antibodies (anti-Rabbbit IgG-HRP; ABClon, Korea) at room temperature for 1 hour, and blots were developed by using ECL (Bio-rad, USA). Results thereof are shown in FIG. 1B.

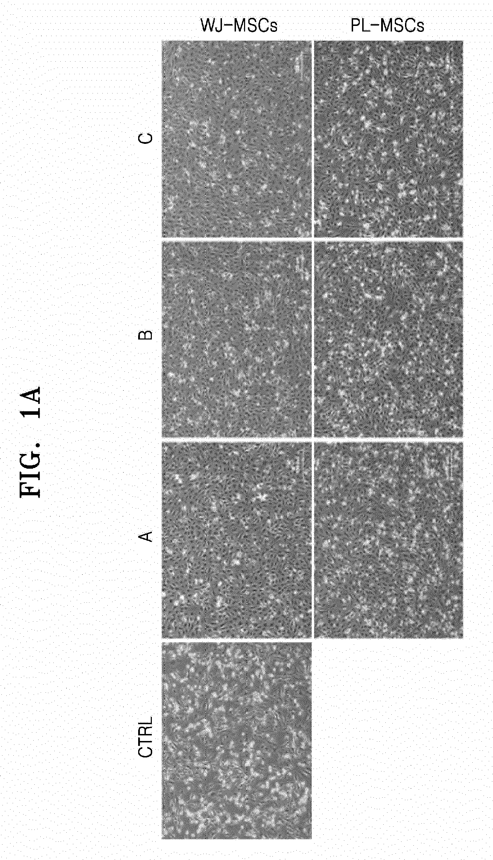

FIG. 1A shows results of confirming the anti-cell death effects of the PL-MSCs and the WJ-MSCs in skeletal muscle cells.

FIG. 1B shows results of the expression of cell death markers by the PL-MSCs and the WJ-MSCs as confirmed through western blotting.

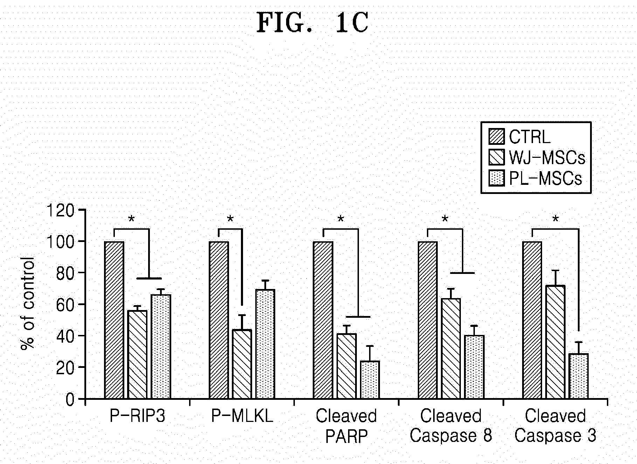

FIG. 1C shows a graph of quantifying the expression of cell death markers by the PL-MSCs and the WJ-MSCs.

Consequently, as shown in FIG. 1A, it was confirmed that, when the cell death-induced C2C12 cells by lovastatin treatment were co-cultured with the PL-MSCs and the WJ-MSCs, the cell death was inhibited. Also, as shown in FIG. 1B, it was confirmed that, when the cell death-induced C2C12 cells by lovastatin treatment were co-cultured with the PL-MSCs and the WJ-MSCs, the expression of apoptosis markers, such as cleaved PARP, cleaved caspase 3, and cleaved caspase 8, and the expression of necrosis markers, such as p-RIP3 and p-MLKL, were inhibited. In particular, it was confirmed that the expression of apoptosis markers was significantly reduced in the group co-cultured with the PL-MSCs, and that the expression of the necrosis markers was significantly reduced in the group co-cultured with the WJ-MSCs. These results were also confirmed in FIG. 1C in which the western blotting results are quantified. In detail, it was confirmed that, compared to the control, the expression of apoptosis markers was decreased by 70 % and the expression of necrosis markers was decreased by 30 % in the group co-cultured with the PL-MSCs. Also, it was confirmed that, compared to the control group, the expression of apoptosis markers and the expression of necrosis markers were decreased by 50 %, respectively, in the group co-cultured with the WJ-MSCs. That is, it was confirmed that the PL-MSCs and the WJ-MSCs inhibited the death of muscle cells by apoptosis and necrosis.

### Example 2. Confirmation of mechanism of inhibiting skeletal muscle cells death through necrosis inhibition

Based on the results of Example 1, proteins secreted from the PL-MSCs and the WJ-MSCs were confirmed under conditions of the death of muscle cells, to confirm a mechanism of inhibiting the death of muscle cells through necrosis inhibition. In detail, skeletal muscle myoblasts of a C2C12 mouse were treated with 10 µm of lovastatin and cultured for 3 hours to induce cell death. Afterwards, the mouse skeletal muscle myoblasts were co-cultured with PL-MSCs and WJ-MSCs, respectively, for 24 hours. After fixing the cultured mouse skeletal muscle myoblasts by using 4 % paraformaldehyde for 15 minutes, the resulting cells were treated with fluorescent reagents each capable of staining apoptic cells (green), necrotic cells (red), and healthy cells (blue) by using an apoptosis/necrosis assay kit, followed by culturing at room temperature for about 1 hour. Then, the cell states were observed under a fluorescence microscope.

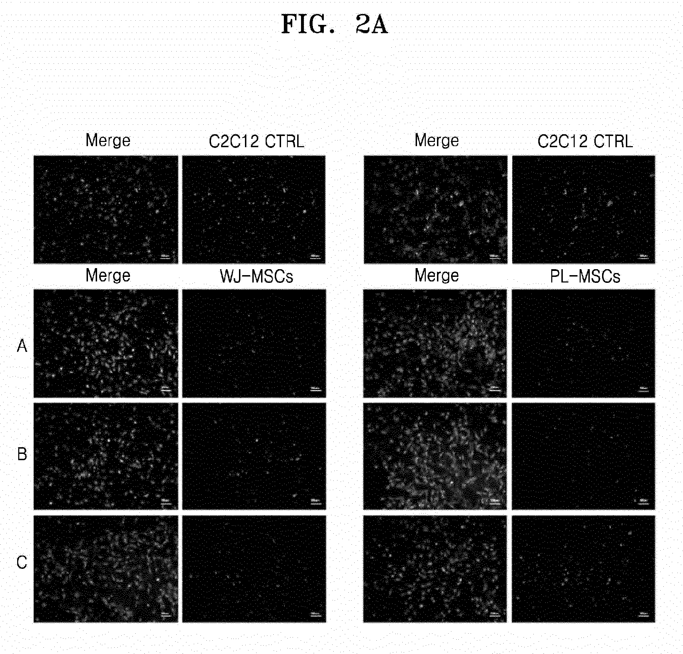

FIG. 2A shows results of confirming the inhibitory effects of the PL-MSCs and the WJ-MSCs on the death of skeletal muscle cells through necrosis inhibition.

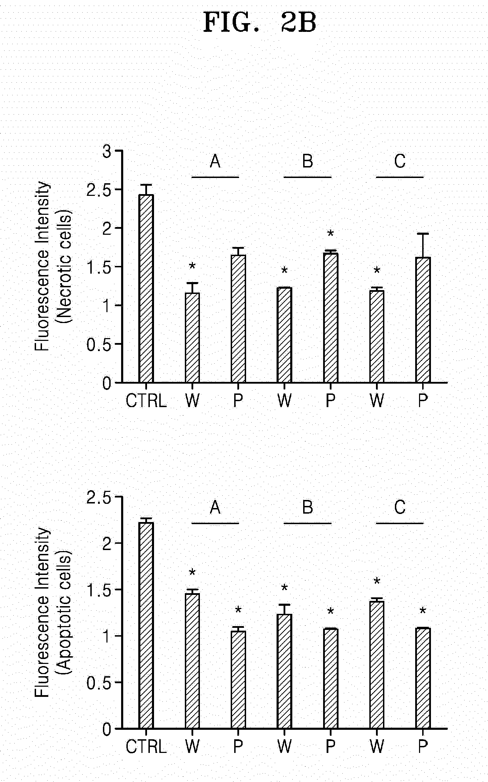

FIG. 2B is a graph of quantifying the states of necrotic cells (left) and apoptic cells (right) in the cell death-induced C2C12 cells that are co-cultured with the PL-MSCs and the WJ-MSCs.

Consequently, as shown in FIG. 2A, it was confirmed that, when the cell death-induced C2C12 cells were co-cultured with the PL-MSCs and the WJ-MSCs, apoptosis and necrosis were all decreased in the cells. In detail, it was confirmed that the number of dead cells by apoptosis was decreased in the group co-cultured with the PL-MSCs, and that the number of dead cells by necrosis was decreased in the group co-cultured with the WJ-MSCs. These results were also confirmed in FIG. 2B in which the intensity of apoptotic cells and necrotic cells is quantified. In detail, it was confirmed that the intensity of apoptic cells was decreased by 2-fold and 0.5-fold in the group co-cultured with the PL-MSCs and the group co-cultured with the WJ-MSCs, respectively, compared to the control. Also, it was confirmed that the intensity of necrotic cells was decreased by 1.5-fold and 2.5-fold in the group co-cultured with the PL-MSCs and the group co-cultured with the WJ-MSCs, respectively, compared to the control.

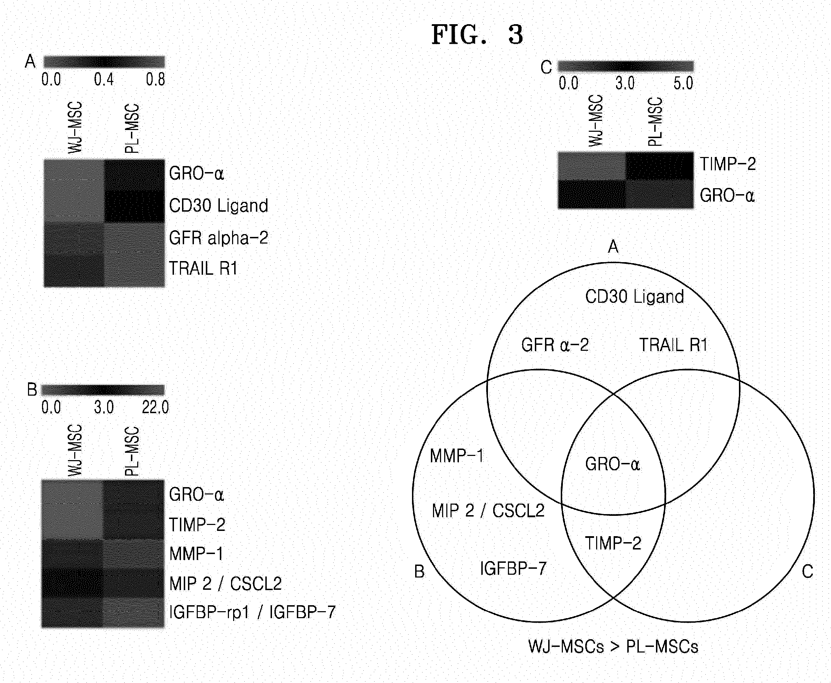

FIG. 3 shows results of analyzing proteins commonly expressed in the PL-MSCs and WJ-MSCs co-cultured with the cell death-induced C2C12 cells.

Consequently, as shown in FIG. 3, it was confirmed that, when the cell death-induced C2C12 cells by lovastatin treatment were co-cultured with the PL-MSCs and the WJ-MSCs, GRO-α proteins were commonly expressed in the cells. Accordingly, it was confirmed that the GRO-α proteins were able to inhibit the cell death.

### Example 3. Confirmation of inhibitory effect of GRO-α on skeletal muscle cell death

### 3-1. Inhibitory effect of GRO-α on death of skeletal muscle myoblasts

In Example 2, the inhibitory effect of GRO-α, which is the protein commonly expressed increasingly in the PL-MSCs and the WJ-MSCs, on the cell death was confirmed by apoptosis markers and necrosis markers. In detail, after co-culturing the cell death-induced mouse skeletal muscle myoblasts with PL-MSCs or WJ-MSCs, each culture medium was collected and concentrated 10 times to confirm changes in 507 various secretory proteins derived from human MSCs by using a RayBio biotin label-based human antibody array kit (#AAH-BLG-1-4). Among these proteins, the secretory proteins commonly expressed increasingly in the WJ-MSCs compared to the PL-MSCs were searched for.

In addition, the cell death-induced mouse skeletal muscle myoblasts by lovastatin treatment were treated with GRO-α diluted to a final concentration of 0 µg/mL, 50 µg/mL, 100 µg/mL, and 200 µg/mL, and then, cultured for 24 hours to observe cell morphology.

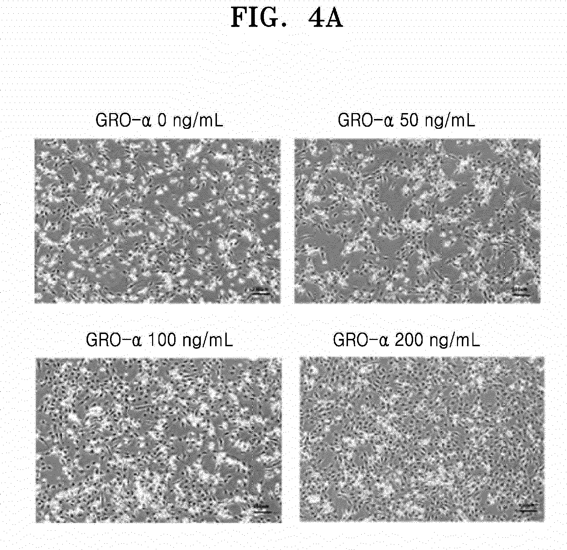

FIG. 4A shows results of the cell morphology after the cell death-induced C2C12 by lovastatin treatment were treated with GRO-α at different concentrations.

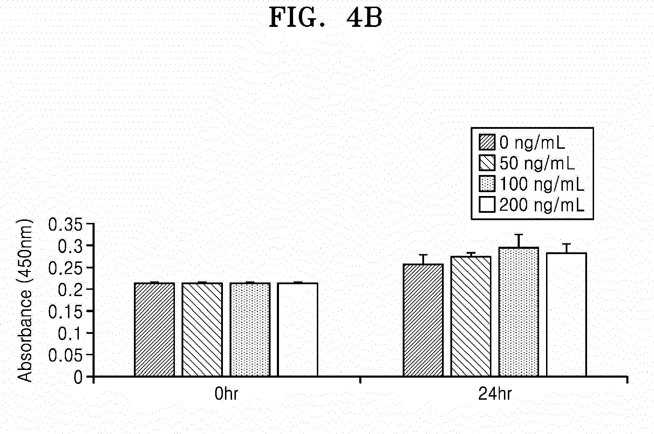

FIG. 4B shows results confirming cell viability after the cell death-induced C2C12 by lovastatin treatment were treated with GRO-α at different concentrations.

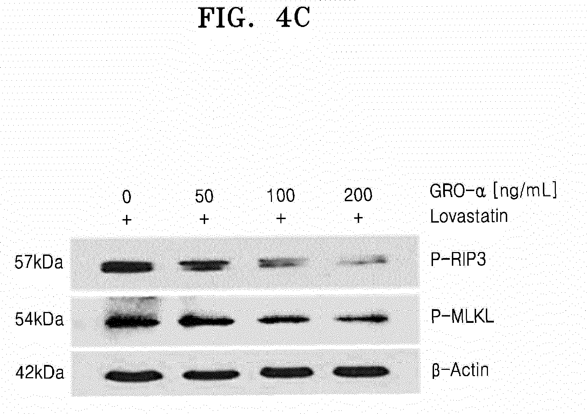

FIG. 4C shows results of expression of the necrosis markers as confirmed through western blotting after the cell death-induced C2C12 by lovastatin treatment were treated with GRO-α at different concentrations.

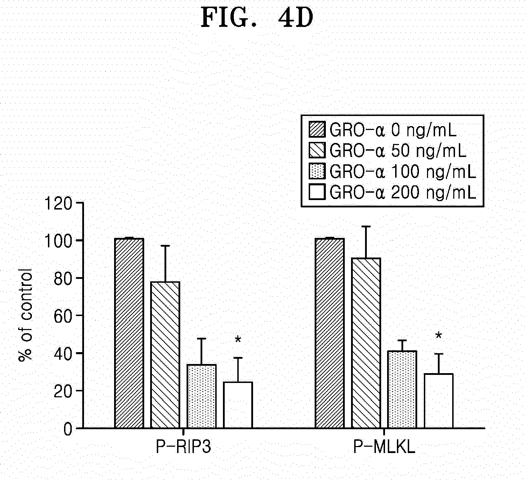

FIG. 4D shows a graph of quantifying the expression of necrosis markers after the cell death-induced C2C12 by lovastatin treatment were treated with GRO-α at different concentrations.

Consequently, as shown in FIG. 4A, it was confirmed that the cells treated with GRO-α inhibited the cell death so that the cell proliferation rate was increased. Also, as shown in FIG. 4B, it was confirmed that the cell viability significantly increased after 24 hours of treatment with GRO-α. In particular, it was confirmed that the cell viability was the highest in the group treated with 100 ng/mL of GRO-α.

Also, as shown in FIGS. 4C and 4D, it was confirmed that GRO-α inhibited the expression of necrosis markers. In particular, it was confirmed that GRO-α inhibited the expression of the necrosis markers in a concentration-dependent manner. That is, it was confirmed that GRO-α inhibited the cell death by inhibiting necrosis in a concentration-dependent manner so that the cell viability was increased.

### 3-2. Comparison of effects of RIP-3 inhibitor and GRO-α on skeletal muscle cell death

Inhibitory effects of GSK'827 and GRO-α, known as apoptosis inhibitors that block the RIP-3 pathway, on cell death of skeletal muscle myoblasts were compared. In detail, skeletal muscle myoblasts of the cell death-induced mouse by lovastatin treatment were pretreated with 3 µm of GSK'827 for 3 hours, followed by washing with 1X PBS. The medium was replaced by a serum-free medium, and GRO-α was diluted therein to a final concentration of 200 µg/mL. Then, the cells were treated with GRO-α and GSK'827 individually or in combination, followed by culturing for 48 to observe the cell morphology.

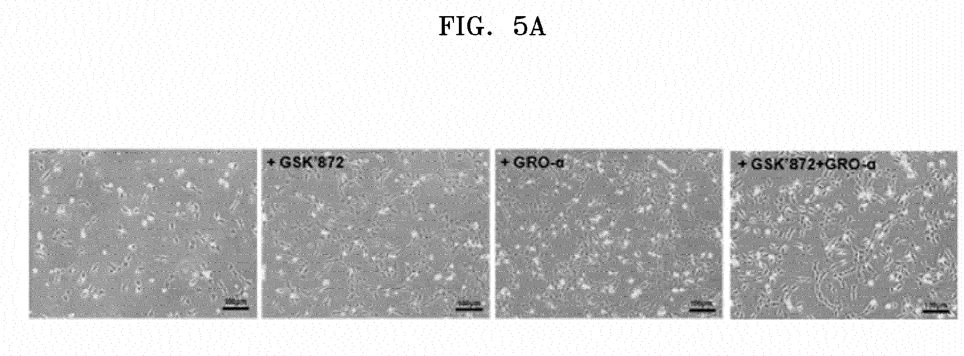

FIG. 5A shows results of cell morphology after the cell death-induced C2C12 cells by lovastatin treatment were treated with GSK'872 and GRO-α individually or in combination.

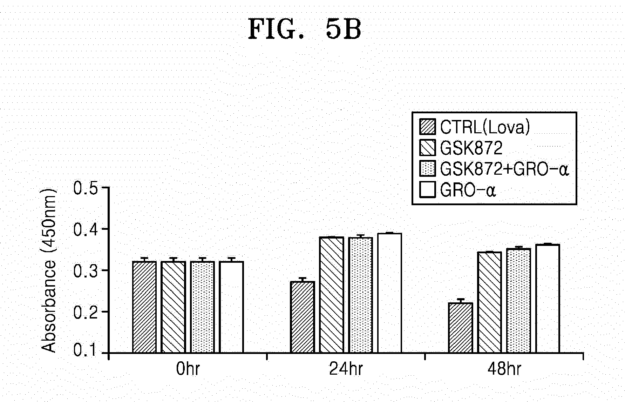

FIG. 5B shows results of cell viability after the cell death-induced C2C12 cells by lovastatin treatment were treated with GSK'872 and GRO-α individually or in combination.

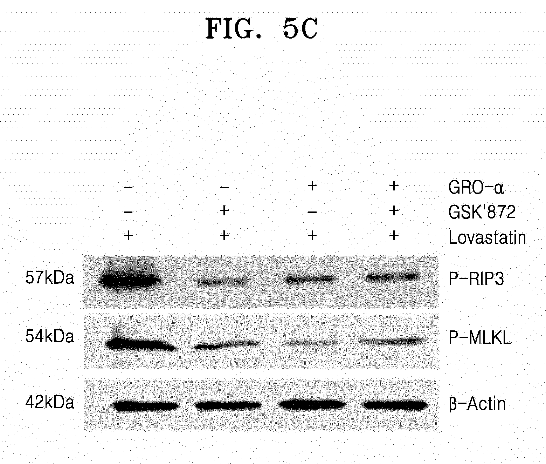

FIG. 5C shows results of expression of necrosis markers confirmed by western blotting after the cell death-induced C2C12 cells by lovastatin treatment were treated with GSK'872 and GRO-α individually or in combination.

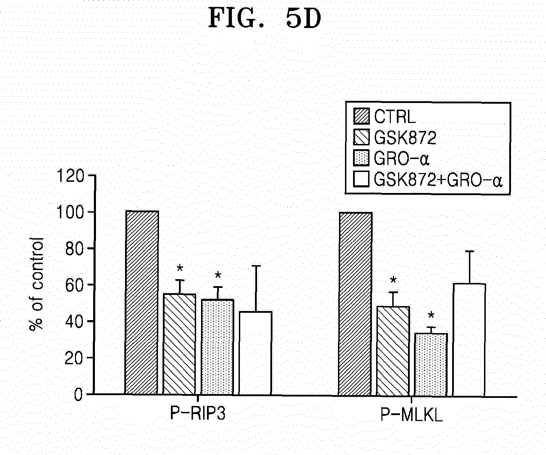

FIG. 5D shows a graph quantifying expression of necrosis markers after the cell death-induced C2C12 cells by lovastatin treatment were treated with GSK'872 and GRO-α individually or in combination.

Consequently, as shown in FIG. 5A, it was confirmed that cell death-induced C2C12 cells by lovastatin treatment were proliferated by the treatment with GSK'872 and/or GRO-α. Also, as shown in FIG. 5B, it was confirmed that cell death-induced C2C12 cells by lovastatin treatment showed a significant increase in cell viability by the treatment with GSK'872 and/or GRO-α. In detail, the cell viability was high in the group treated with GRO-α compared to the group treated with GSK'872, and when compared to the cells treated with GSK'872 and GRO-α in combination, the same results were confirmed.

Also, as shown in FIGS. 5C and 5D, GRO-α reduced the expression of all markers involved in necrosis compared to the control group, and accordingly, it was confirmed that, compared to GSK'872, GRO-α had an excellent inhibitory effect on the expression of necrosis markers. Therefore, by inhibiting the cell death of muscle cells, GRO-α is available as a therapeutic agent for preventing or treating various muscle diseases.

### Example 4. Confirmation of in vivo activity

### 4-1. Confirmation of inhibition on activity of creatine kinase.

Based on the results of Examples 1 to 3, the efficacy of GRO-α on prevention or treatment of muscle diseases was confirmed in an MDX mouse (a mouse model for duchenne muscular dystrophy). In detail, the MDX mouse was intravenously and intraperitoneally injected with each of WJ-MSC and GRO-α. After one week, blood was collected from the heart of the mouse, centrifuged at 15,500 X g for 10 minutes to separate serum, and then, activity of creatine kinase was measured.

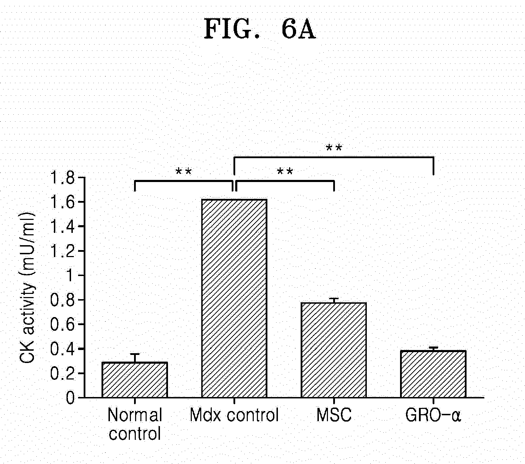

FIG. 6A shows a graph of measuring the activity of creatine kinase in the blood of the MDX mouse injected with WJ-MSCs and GRO-α.

Consequently, as shown in FIG. 6A, it was confirmed that, compared to a negative control, the activity of creatine kinase increased about 8 times in the MDX mouse, and by the injection of WJ-MSCs and GRO-α, the activity of creatine kinase decreased by 50 % and 75 %, respectively.

That is, GRO-α and the mesenchymal stem cells expressing the same according to an aspect can be used for the prevention or treatment of muscle diseases by significantly reducing the activity of creatine kinase which is a biomarker for muscle loss.

### 4-2. Confirmation of inhibition of necrosis

The expression levels of necrosis markers in muscles of the MDS mouse injected with WJ-MSCs and GRO-α were confirmed in in Example 4-1. In detail, the muscle tissue of the mouse was ground with liquid nitrogen into a minimum size in a mortar, added to an E-tube, and then subjected to sonication with lysis buffer(urea+RIPA=1:1). Afterwards, the supernatant was collected, and proteins in the tissue were quantified according to the Bradford assay (Bio-rad, USA). Then, proteins were detected by using western blot.

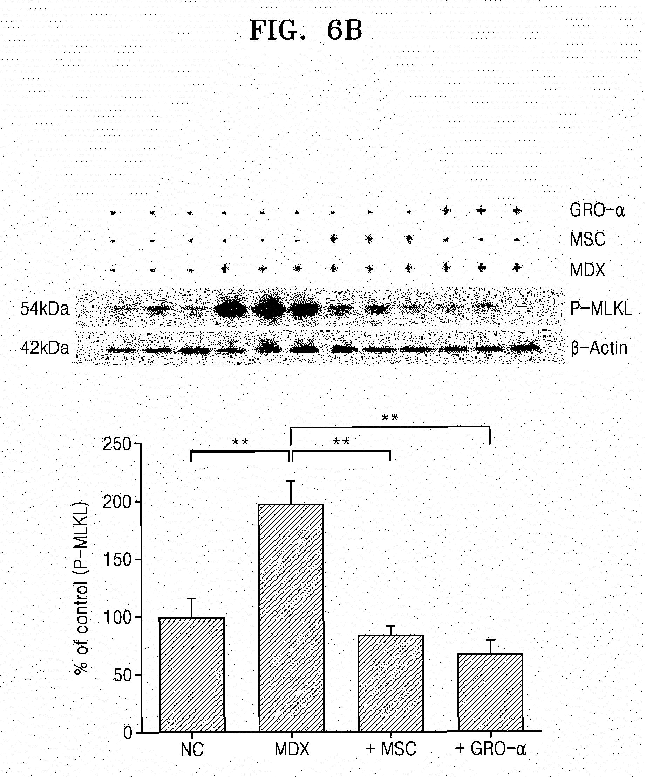

FIG. 6B shows results of measuring, at a protein level, expression levels of phospho-MLKL in the muscles of the mouse injected with WJ-MSCs and GRO-α.

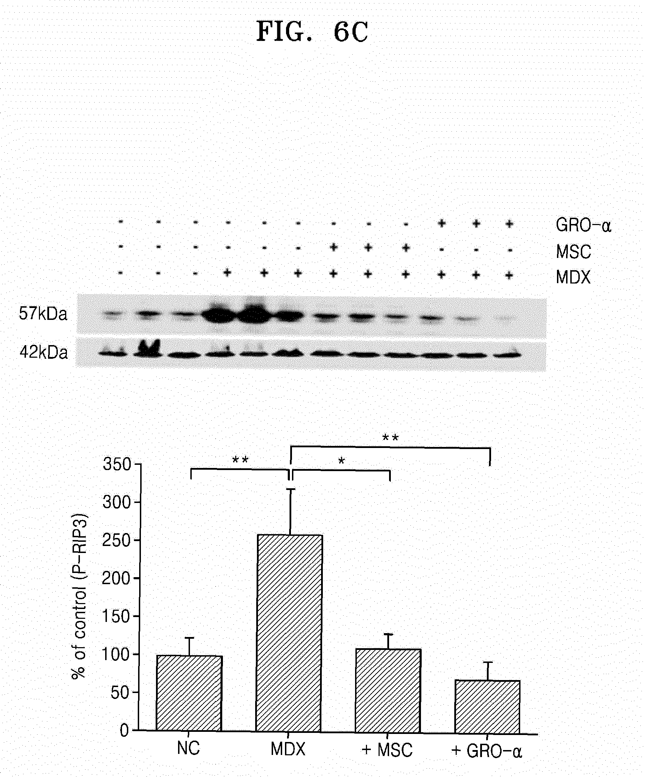

FIG. 6C shows results of measuring, at a protein level, expression levels of phospho-RIP3 in the muscles of the MDX mouse injected with WJ-MSCs and GRO-α.

Consequently, as shown in FIG. 6B, it was confirmed that, compared to a negative control, the expression of phospho-MLKL increased about 2 times to 2.5 times in the MDX mouse, and by the injection of WJ-MSCs and GRO-α, the expression of phospho-MLKL decreased by 60 % and 76 %, respectively. Also, as shown in FIG. 6C, it was confirmed that, compared to a negative control, the expression of phospho-RIP3 increased about 2 times to 2.5 times in the MDX mouse, and by the injection of WJ-MSCs and GRO-α, the expression of phospho-RIP3 decreased by 60 % and 70 %, respectively.

That is, GRO-α and the mesenchymal stem cells expressing the same according to an aspect can inhibit the cell death of muscle cells by reducing the expression of necrosis markers in muscle, and thus can be used for the prevention or treatment of various muscle diseases.

The foregoing descriptions are only for illustrating the present disclosure, and it will be apparent to a person having ordinary skill in the art to which the present invention pertains that the embodiments disclosed herein can be easily modified into other specific forms without changing the technical spirit or essential features. Therefore, it should be understood that Examples described herein are illustrative in all respects and are not limited.

## Claims

1. A cell death inhibitor comprising: a mesenchymal stem cell genetically engineered to secrete growth-regulated oncogene-alpha (GRO-α) or overexpress GRO-α compared to a parent cell; GRO-α, or a combination thereof.

2. The cell death inhibitor of claim 1, wherein the mesenchymal stem cell is umbilical cord-derived, umbilical cord blood-derived, bone marrow-derived, placenta-derived, or adipose-derived.

3. A pharmaceutical composition for preventing or treating a muscle disease, the pharmaceutical composition comprising: a mesenchymal stem cell genetically engineered to secrete growth-regulated oncogene-alpha (GRO-α) or overexpress GRO-α compared to a parent cell; GRO-α; or a combination thereof.

4. The pharmaceutical composition of claim 3, wherein the muscle disease is selected from the group consisting of sprain, strain, spasm, tendinitis, muscle cancer, myositis, rhabdomyolysis, muscular atrophy, atony, muscular dystrophy, muscular degeneration, myasthenia, dystrophinopathy, myopathy, sacopenia, and Charot-Marie-Tooth disease.

5. A pharmaceutical composition for preventing or treating a muscle disease, the pharmaceutical composition comprising growth-regulated oncogene-alpha (GRO-α) as an active ingredient.

6. The pharmaceutical composition of claim 5, further comprising a mesenchymal stem cell.

7. The pharmaceutical composition of claim 6, wherein GRO-α is separated from the mesenchymal stem cell.

8. A health functional food composition for preventing or treating a muscle disease, the health functional food composition comprising growth-regulated oncogene-alpha (GRO-α) as an active ingredient.

9. A cell therapy agent for treating a muscle disease, the cell therapy agent comprising: a mesenchymal stem cell genetically engineered to secrete growth-regulated oncogene-alpha (GRO-α) or overexpress GRO-α compared to a parent cell; GRO-α; or a combination thereof.

10. A method of preventing or treating a muscle disease, the method comprising administering a pharmaceutically effective amount of a mesenchymal stem cell, which is genetically engineered to secrete growth-regulated oncogene-alpha (GRO-α) or overexpress GRO-α compared to a parent cell, or GRO-α, to a subject in need thereof.

11. Use of a composition for treating a muscle disease, the composition comprising: a mesenchymal stem cell genetically engineered to secrete growth-regulated oncogene-alpha (GRO-α) or overexpress GRO-α compared to a parent cell; or GRO-α.

12. Use of a composition for preparing a therapeutic agent for a muscle disease, the composition comprising: a mesenchymal stem cell genetically engineered to secrete growth-regulated oncogene-alpha (GRO-α) or overexpress GRO-α compared to a parent cell; or GRO-α.
